# EUROPEAN PATENT SPECIFICATION

(11) **EP 0 619 119 B1**
(45) Date of publication and mention of the grant of the patent: **31.03.1999**
(21) Application number: 92924889.6
(22) Date of filing: 08.12.1992
(51) Int. Cl.: A61K 38/29, A61K 47/26, A61K 47/02

(54) **STABILIZED PARATHYROID HORMONE COMPOSITION**
STABILISIERTE PARATHORMONZUSAMMENSETZUNG
COMPOSITION A BASE D'HORMONE PARATHYROIDIENNE STABILISEE

(30) Priority: 09.12.1991 JP 350236/91; 24.11.1992 JP 313381/92
(43) Date of publication of application: 12.10.1994
(73) Proprietor: Asahi Kasei Kogyo Kabushiki Kaisha, Osaka-shi, Osaka 530-8205 (JP)
(72) Inventor: ENDO, Ken 163-1, Daiba, Shizuoka 411 (JP); SATO, Shinji 23-8, Nishizoecho, Shizuoka 410-03 (JP); SUGIYAMA, Yoshinori 1486-1, Kamikanuki Futasegawa, Shizuoka 410 (JP); OHNO, Masaru 339-14, Takyo Ohito-cho, Shizuoka 410-23 (JP); SAKAKIBARA, Hideo 273-12, Naka, Shizuoka 411 (JP)
(74) Representative: Boeters, Hans Dietrich, Dr.
(86) International application number: PCT/JP92/01599
(87) International publication number: WO 93/11785

(56) References cited:
- JP-A- 2 022 233
- JP-A- 3 041 033
- JP-A- 4 247 034
- JP-A-63 060 940

## Description

### FIELD OF THE INVENTION:

The present invention relates to a method for stabilizing parathyroid hormone (hereinafter generally designates as PTH) and a composition containing parathyroid hormone as the active ingredient.

### PRIOR ARTS:

PTH is a hormone, of which the same as of calcitonin and vitamin D groups, which pertains controlling serum calcium level and is used as a diagnostic reagent for hypoparathyroidism. In a lyophilized preparation for injectable solution at use containing trace amount of PTH, a sugar such as mannitol or polymer substance such as gelatin is generally combined. (Japanese Patent Unexamined Publication No. 63-60940 and ibid., No. 2-111)

### PROBLEMS TO BE SOLVED BY THE INVENTION:

Mannitol is used conventional stabilising agent for the lyophilized preparation of peptide or protein for injectable solution at use. However, when mannitol is used as a stabilizing agent for the lyophilized preparation of PTH for injectable solution at use, its stabilizing effect is ended an unsatisfactory result. Polymer substance such as gelatin gives high stabilizing effect, however its immunological safety problem is still uncertainly remained.

### MEANS FOR SOLVING PROBLEMS:

We have continued to study for finding a stable preparation of PTH, and an unexpected discovery was made during the study that extremely more improved stability for the lyophilized preparation of PTH has been obtained by combining a constant amount of sodium chloride in sugar together with lyophilization, as compared with sugar alone.

As a result, a safety with excellent heat stable lyophilized preparation of PTH can be prepared by adding a combination of sugar and sodium chloride as a stabilizing agent for lyophilized preparation of PTH.

An object of the present invention is to provide a lyophilized preparation of PTH containing PTH as the active ingredient and effective amounts of a sugar and sodium chloride as the stabilizing agent.

Another object of the present invention is to provide a method for stabilizing a preparation of PTH comprises dissolving PTH, effective amounts of a sugar and sodium chloride in an aqueous medium, and lyophilizing the same.

An active ingredient PTH is a peptide possessing a serum calcium increasing activity, with a molecular weight of approx. 4000 - 10000, consisting of an amino acid sequence of 34-84 amino acids, and natural PTH or its analogue are known. Examples thereof are human PTH(h-PTH)(1-84)[Biochemistry, 17, 5723 (1978)], h-PTH(1-38) (Japanese Patent Unexamined Publication No. 57-81448), h-PTH(1-34)[Hoppe Seyler's Z. Physiol. Chem., 355, 415 (1974)], h-PTH(1-34)NH₂ (Japanese Patent Unexamined Publication No. 58-96052),[Nle^{8,18}]h-PTH(1-34), [Nle^{8,18},Tyr³⁴]h-PTH(1-34) (ibid., No.55-113753), [Nle^{8,18}]h-PTH(1-34)NH₂(ibid., No.61-24598), [Nle^{8,18},Tyr³⁴]h-PTH(1-34)NH₂(ibid., No.60-24996), rat-PTH(1-84)[J. Biol. Chem., 259(5), 3320 (1984)], rat-PTH(1-34) [Endocrinol., 117(3), 1230(1985)], bovine-PTH(1-84)[Am. J. Med., 50, 639(1971)], bovine -PTH(1-34) and bovine-PTH(1-34)NH₂[Phytho biology Annual, 11, 53 (1981)].

Preferable example is a h-PTH(1-34) of molecular weight approx. 4400, with amino acid sequence of 34 amino acids.

Examples of sugar in the present invention are, preferably a monosaccharide or disaccharide. Examples of monosaccharide are mannitol, glucose, sorbitol or inositol, and among them mannitol is most preferable. Examples of disaccharides are sucrose, maltose, lactose and treharose. These disaccharides can be used alone or in combination with more than two kinds of sugar. Most preferable examples of disaccharide are sucrose, maltose and lactose.

Amount of sugars to be added is preferably more than one weight part of sugar for one weight part of PTH, and 50 - 1000 weight part of sugar for one weight part of PTH is more preferable.

The larger amount of sodium chloride to be added for that of sugar, the more stable for PTH. However in the case that amount of sodium chloride exceeds 20% weight for the amount of sugar, lyophilized cake of the preparation become shrinking and the stability shows tendency of decrease, so that the amount of sodium chloride to be added is 1/1000 weight part to 1/5 weight part for one weight part of sugar, preferable 1/100 - 1/10 weight part thereof.

Examples of aqueous medium are distilled water for injection, physiological saline or buffer solution. Water soluble organic solvent such as nontoxic amount of ethanol can be added to the above aqueous medium.

The lyophilized composition of the present invention, can be prepared by that, for example PTH, sugar and sodium chloride of the above composition, and if required known pH adjuster, isotonicity agent, stabilizing agent, excipient and antiseptic are mixed thereto, and the mixture is dissolved in the above aqueous medium, and after aseptic filtration lyophilized with conventional means. Lyophilization can be made by conventional condition with lyophilization on tray lyophilization or lyophilization in vial .

The thus prepared lyophilized composition can be formulated by the dosage form design containing, for example, 1 µg- 150 µg of PTH, preferably such as h-PTH(1-34), in an injectable lyophilized preparation.

### EXAMPLES:

### Example 1.

An h-PTH(1-34)(Asahi Chemical Industry Co.) 2.16mg, mannitol 300mg and sodium chloride 30mg were dissolved in sterile distilled water 30 ml. After aseptic filtration, each 0.5 ml of the solution was separately poured into glass vials, lyophilized, replaced with nitrogen atmosphere, fully inserted stopper and applied the aluminum seal to prepare lyophilized preparation for injectable solution at use. (hereinafter designates as the preparation 1)

### Example 2.

In example 1, mannitol was replaced by the sugars indicated in the following table 1 together with adding sodium chloride of which amount shown in the table 1, and others were treated as same as of in example 1 to prepare the lyophilized preparation as indicated.

**Table 1**

| the preparation | sugar | | sodium chloride |
|---|---|---|---|
| 2 | sucrose | 300 mg | 6 mg |
| 3 | sucrose | 300 mg | 30 mg |
| 4 | sucrose | 300 mg | 60 mg |
| 5 | sucrose | 600 mg | 60 mg |
| 6 | maltose | 300 mg | 30 mg |
| 7 | lactose | 300 mg | 30 mg |
| 8 | sucrose | 600 mg | 30 mg |
| 9 | mannitol | 600 mg | 30 mg |

### Example 3.

Bovine PTH(1-34)(Sigma corp.) 0.3 mg, mannitol 240 mg and sodium chloride 24 mg were dissolved in sterile distilled water 12 ml. After aseptic filtration each 0.5 ml of the solution was separately poured into glass ampules, lyophilized and fuse-sealed to prepare the preparation for injectable solution at use.

### Example 4.

Rat PTH(1-34)(Asahi Chem. Ind. Co.) 0.2 mg, trehalose 160 mg and sodium chloride 10 mg were dissolved in sterile distilled water 8 ml. Each 0.1 ml of the solution was separately poured into glass ampules, lyophilized and fuse-sealed to prepare a reference standard for measuring the content.

### Example 5.

[Nle^{8,18}]-h-PTH(1-34) (Asahi Chemical Industry Co.) 0.6 mg, trehalose 160 mg and sodium chloride 24 mg were dissolved in sterile distilled water 24 ml. After aseptic filtration, each 0.5 ml of the solution was separately poured into glass vials, lyophilized, fully inserted stopper and applied the aluminum seal to prepare lyophilized preparation for injectable solution at use.

### Example 6.

h-PTH(1-84)(Sigma Corp.) 0.15mg, trehalose 100 mg and sodium chloride 10 mg were dissolved in sterile distilled water 10 ml. Each 0.2 ml of the solution was separately poured into glass ampules, lyophilized and fuse-sealed to prepare a reference standard for assaying the biological activity.

### Example 7.

h-PTH(1-34) (Asahi Chemical Industry Co.) 0.54 mg, glucose 75 mg and sodium chloride 7.5 mg were dissolved in sterile distilled water 15 ml. After aseptic filtration, each 1 ml of the solution was separately poured into glass vials, lyophilized, fully inserted stopper and applied the aluminum seal to prepare lyophilized preparation for injectable solution at use.

### Example 8.

h-PTH(1-34) (Asahi Chemical Industry Co.) 1.08 mg, sorbitol 300 mg and sodium chloride 15 mg were dissolved in sterile distilled water 15 ml. After aseptic filtration, each 0.5 ml of the solution was separately poured into glass vials, lyophilized, fully inserted stopper and applied to prepare lyophilized preparation for injectable solution at use.

### Example 9.

h-PTH(1-34) (Asahi Chemical Industry Co.) 1.08 mg, inositol 300 mg and sodium chloride 30 mg were dissolved in sterile distilled water 15 ml. After aseptic filtration, each 0.5 ml of the solution was separately poured into glass vials, lyophilized, fully inserted stopper and applied the aluminum seal to prepare lyophilized preparation for injectable solution at use.

### Example 10.

### Stability test for PTH:

### [Test materials]

The preparations 1 - 9 obtained in examples 1 and 2, and the control preparations 1 - 7 hereinbelow were used for stability test.

### [Preparing the control preparation]

(1) An h-PTH(1-34) (Asahi Chemical Industry Co.) 2.16 mg and sodium chloride 30 mg were dissolved in sterile distilled water 30 ml. After aseptic filtration, each 0.5 ml of the solution was separately poured into glass vials, lyophilized, replaced with nitrogen atmosphere, fully inserted stopper and applied the aluminum seal to prepare lyophilized preparation for injectable solution at use. (the control preparation 1)
(2) An h-PTH(1-34) (Asahi Chemical Industry Co.) 2.16 mg and mannitol 300 mg were used and the other operation was treated as the same as of the above to prepare the preparation for injectable solution at use. (the control preparation 2)
(3) An h-PTH(1-34) (Asahin Chemical Industry Co.) 2.16 mg and sucrose 300 mg were used and the other operation was treated as the same as of the above to prepare the preparation for injectable solution at use. (the control preparation 3)
(4) An h-PTH(1-34) (Asahi Chemical Industry Co.) 2.16 mg and maltose 300 mg were used and the other operation was treated as the same as of the above to prepare the preparation for injectable solution at use. (the control preparation 4)
(5) An h-PTH(1-34) (Asahin Chemical Industry Co.) 2.16 mg and lactose 300 mg were used and the other operation was treated as the same as of the above to prepare the preparation for injectable solution at use. (the control preparation 5)
(6) An h-PTH(1-34) (Asahi Chemical Industry Co.) 2.16 mg and sucrose 600 mg were used and the other operation was treated as the same as of the above to prepare the preparation for injectable solution at use. (the control preparation 6)
(7) An h-PTH(1-34) (Asahin Chemical Industry Co.) 2.16 mg and mannitol 600 mg were used and the other operation was treated as the same as of the above to prepare the preparation for injectable solution at use. (the control preparation 7)

### [Test method]

The preparations 1 - 9 obtained in the examples 1 and 2, and the control preparations 1 - 7 were stored at 40 °C. Each sample was collected at constant time passed and PTH content thereof was measured by means of high performance liquid chromatography (HPLC) under the following conditions.

### HPLC measuring condition

Column: YMC AM-303 ODS S-5 120Å (YMC Co.) inner diameter 4.6 × 250 mm
Mobile phase: 0.1% TFA : acetonitrile = 73:27-68:32(17 min.)
Flow rate: 1.0 ml/min.
Detection: UV 280 nm

### [Test result]

A composition of each sample and residual rate of PTH within 3 months at 40 °C are shown in Table 2.

**Table 2**

| Sample | Sugar | Amount added* | Sodium chloride | PTH residual rate (%) at 40°C | | | |
|---|---|---|---|---|---|---|---|
| | | | | initial | 1M ** | 2M ** | 3M ** |
| control 1 | no add. | | 5.0 mg | 100 | 86 | 82 | 75 |
| control 2 | mannitol | 5 mg | no addition | 100 | 89 | 84 | 76 |
| preparation 1 | mannitol | 5 mg | 0.5 mg | 100 | 94 | 93 | 91 |
| control 3 | sucrose | 5 mg | no addition | 100 | 90 | 85 | 74 |
| preparation 2 | sucrose | 5 mg | 0.1 mg | 100 | 95 | 93 | 90 |
| preparation 3 | sucrose | 5 mg | 0.5 mg | 100 | 98 | 95 | 93 |
| preparation 4 | sucrose | 5 mg | 1.0 mg | 100 | 96 | 93 | 90 |
| preparation 5 | sucrose | 10 mg | 1.0 mg | 100 | 99 | 97 | 95 |
| control 4 | maltose | 5 mg | no addition | 100 | 84 | 79 | 73 |
| preparation 6 | maltose | 5 mg | 0.5 mg | 100 | 95 | 94 | 92 |
| control 5 | lactose | 5 mg | no addition | 100 | 83 | 80 | 74 |
| preparation 7 | lactose | 5 mg | 0.5 mg | 100 | 94 | 92 | 91 |
| control 6 | sucrose | 10 mg | no addition | 100 | 89 | 84 | 79 |
| preparation 8 | sucrose | 10 mg | 0.5 mg | 100 | 98 | 96 | 96 |
| control 7 | mannitol | 10 mg | no addition | 100 | 86 | 82 | 75 |
| preparation 9 | mannitol | 10 mg | 0.5 mg | 100 | 91 | 90 | 89 |

| | | | | | | | |
|---|---|---|---|---|---|---|---|
| * amount added is shown by a conversion of numerical value per vial. | | | | | | | |
| ** month | | | | | | | |

As shown in Table 2, the preparation of the present invention which contains combination of sodium chloride and sugar is more stable than the control preparation containing sodium chloride alone or sugar alone, and the effect of combination of sodium chloride and sugar was confirmed. An addition of sugar alone reveals extremely inferior stability at 3 months storage at 40 °C.

Further addition of sodium chloride alone shows disadvantages of shrinking the lyophilized cake and decreased stability.

### EFFECT OF THE INVENTION:

As illustrated, a highly safe, stabilized lyophilized preparation of a parathyroid hormone (PTH) containing PTH as the active ingredient and effective amounts of a sugar and sodium chloride as the stabilizing agents for PTH can be obtained.

## Claims

1. A lyophilized composition comprising parathyroid hormone as the active ingredient and effective amounts of a sugar and sodium chloride as the stabilizing agents for parathyroid hormone.

2. A composition according to claim 1 wherein the sugar is monosaccharide or disaccharide.

3. A composition according to claim 2 wherein the monosaccharide is mannitol.

4. A composition according to claim 2 wherein the monosaccharide is glucose.

5. A composition according to claim 2 wherein the monosaccharide is sorbitol.

6. A composition according to claim 2 wherein the monosaccharide is inositol.

7. A composition according to claim 2 wherein the disaccharide is sucrose.

8. A composition according to claim 2 wherein the disaccharide is maltose.

9. A composition according to claim 2 wherein the disaccharide is lactose.

10. A composition according to claim 2 wherein the disaccharide is treharose.

11. A method for stabilizing parathyroid hormone comprises dissolving parathyroid hormone and effective amounts of a sugar and sodium chloride in an aqueous medium, and lyophilizing the same.

12. A method according to claim 11 wherein the sugar is monosaccharide or disaccharide.

13. A method according to claim 12 wherein the monosaccharide is mannitol.

14. A method according to claim 12 wherein the monosaccharide is glucose.

15. A method according to claim 12 wherein the monosaccharide is sorbitol.

16. A method according to claim 12 wherein the monosaccharide is inositol.

17. A method according to claim 12 wherein the disaccharide is sucrose.

18. A method according to claim 12 wherein the disaccharide is maltose.

19. A method according to claim 12 wherein the disaccharide is lactose.

20. A method according to claim 12 wherein the disaccharide is treharose.

## Patentansprüche

1. Lyophilisierte Zusammensetzung, die Parathormon als Wirkstoff und wirksame Mengen eines Zuckers und von Natriumclorid als Stabilisierungsmittel für Parathormon umfaßt.

2. Zusammensetzung nach Anspruch 1, worin der Zucker ein Monosaccharid oder Disaccharid ist.

3. Zusammensetzung nach Anspruch 2, worin das Monosaccharid Mannitol ist.

4. Zusammensetzung nach Anspruch 2, worin das Monosaccharid Glukose ist.

5. Zusammensetzung nach Anspruch 2, worin das Monosaccharid Sorbitol ist.

6. Zusammensetzung nach Anspruch 2, worin das Monosaccharid Inositol ist.

7. Zusammensetzung nach Anspruch 2, worin das Disaccharid Sucrose ist.

8. Zusammensetzung nach Anspruch 2, worin das Disaccharid Maltose ist.

9. Zusammensetzung nach Anspruch 2, worin das Disaccharid Lactose ist.

10. Zusammensetzung nach Anspruch 2, worin das Disaccharid Trehalose ist.

11. Verfahren zur Stabilisierung von Parathormon, das das Lösen von Parathormon und wirksamen Mengen eines Zuckers und von Natriumclorid in einem wässerigen Medium und Lyophilisation desselben umfaßt.

12. Verfahren nach Anspruch 11, worin der Zucker ein Monosaccharid oder Disaccharid ist.

13. Verfahren nach Anspruch 12, worin das Monosaccharid Mannitol ist.

14. Verfahren nach Anspruch 12, worin das Monosaccharid Glukose ist.

15. Verfahren nach Anspruch 12, worin das Monosaccharid Sorbitol ist.

16. Verfahren nach Anspruch 12, worin das Monosaccharid Inositol ist.

17. Verfahren nach Anspruch 12, worin das Disaccharid Sucrose ist.

18. Verfahren nach Anspruch 12, worin das Disaccharid Maltose ist.

19. Verfahren nach Anspruch 12, worin das Disaccharid Lactose ist.

20. Verfahren nach Anspruch 12, worin das Disaccharid Trehalose ist.

## Revendications

1. Composition lyophilisée comprenant de l'hormone parathyroïdienne comme ingrédient actif et des quantités efficaces de sucre et de chlorure de sodium comme agents stabilisants pour l'hormone parathyroïdienne.

2. Composition selon la revendication 1, dans laquelle le sucre est un monosaccharide ou un disaccharide.

3. Composition selon la revendication 2, dans laquelle le monosaccharide est le mannitol.

4. Composition selon la revendication 2, dans laquelle le monosaccharide est le glucose.

5. Composition selon la revendication 2, dans laquelle le monosaccharide est le sorbitol.

6. Composition selon la revendication 2, dans laquelle le monosaccharide est l'inositol.

7. Composition selon la revendication 2, dans laquelle le disaccharide est le saccharose.

8. Composition selon la revendication 2, dans laquelle le disaccharide est le maltose.

9. Composition selon la revendication 2, dans laquelle le disaccharide est le lactose.

10. Composition selon la revendication 2, dans laquelle le disaccharide est le tréhalose.

11. Procédé de stabilisation de l'hormone parathyroïdienne comprenant le fait de dissoudre l'hormone parathyroïdienne et des quantités efficaces d'un sucre et de chlorure de sodium dans un milieu aqueux, et de lyophiliser ce milieu.

12. Procédé selon la revendication 11, dans lequel le sucre est un monosaccharide ou un disaccharide.

13. Procédé selon la revendication 12, dans lequel le monosaccharide est le mannitol.

14. Procédé selon la revendication 12, dans lequel le monosaccharide est le glucose.

15. Procédé selon la revendication 12, dans lequel le monosaccharide est le sorbitol.

16. Procédé selon la revendication 12, dans lequel le monosaccharide est l'inositol.

17. Procédé selon la revendication 12, dans lequel le disaccharide est le saccharose.

18. Procédé selon la revendication 12, dans lequel le disaccharide est le maltose.

19. Procédé selon la revendication 12, dans lequel le disaccharide est le lactose.

20. Procédé selon la revendication 12, dans lequel le disaccharide est le tréhalose.
